(19) Europäisches Patentamt
European Patent Office
Office européen des brevets

(11) Veröffentlichungsnummer: **0 348 780**
**A2**

(12) # EUROPÄISCHE PATENTANMELDUNG

(21) Anmeldenummer: **89111088.4**

(51) Int. Cl.⁴: **C07K 3/10 , //C12N15/26**

(22) Anmeldetag: **19.06.89**

(30) Priorität: **30.06.88 DE 3822045**

(43) Veröffentlichungstag der Anmeldung:
**03.01.90 Patentblatt 90/01**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Habermann, Paul, Dr.**
**Rossertstrasse 35**
**D-6239 Eppstein/Taunus(DE)**

(54) **Verfahren zur Herstellung von Proteinen.**

(57) Proteine aus gentechnisch kodierten Aminosäuren mit definiertem N-Terminus können unter milden enzymatischen Bedingungen hergestellt werden, wenn man zunächst ein Vorprodukt herstellt, das N-terminal mindestens um Prolin, vorzugsweise um Met-Pro, verlängert ist, worauf - gegebenenfalls nach vorheriger Abspaltung der anderen Aminosäuren mit Aminopeptidase-P - das gewünschte Protein mit Prolin-Iminopeptidase freigesetzt wird. Das Verfahren eignet sich besonders gut für Produkte aus der Direktexpression aus Bakterien, die gegebenenfalls nur teilweise methionisiert sind.

EP 0 348 780 A2

## Verfahren zur Herstellung von Proteinen

Bei der Expression von Proteinen in bakteriellen Systemen werden in der Regel Proteine erhalten, die N-terminal um Methionin verlängert sind. Bei physiologisch wirksamen Proteinen kann dieses zusätzliche Methionin eine Immunogenität bewirken. Zur Vermeidung dieser N-terminalen Verlängerung werden deshalb Gene für Fusionsproteine konstruiert, deren unerwünschter Anteil von dem gewünschten Protein chemisch oder enzymatisch abgespalten werden kann. Die Abtrennung des gewünschten Proteins vom Nebenprodukt oder von den Nebenprodukten kann jedoch sehr aufwendig sein und die Ausbeute reduzieren.

Es gelingt zwar in manchen Fällen, die N-terminale Verlängerung um Methionin bei der Direktexpression aus Bakterien durch geeignete Fermentationsbedingungen mehr oder weniger zu unterdrücken, wobei jedoch die Des-Met-Form nicht quantitativ erhalten wird. Selbst wenn man auf die methionierte Form verzichtet und damit schon eine reduzierte Ausbeute in Kauf nimmt, ist in der Regel doch die Abtrennung des gewünschten Proteins von der methionierten Form außerordentlich schwierig und mit drastischen weiteren Ausbeuteverlusten verbunden.

Es wurde bereits ein Verfahren zur Herstellung von Proteinen vorgeschlagen, die N-terminal mit Prolin beginnen, das dadurch gekennzeichnet ist, daß ein gentechnisch gewonnenes Vorprodukt mit einer kurzen N-terminalen Verlängerung, die vorzugsweise aus einem Methioninrest besteht, der enzymatischen Spaltung mit Aminopeptidase-P unterworfen wird (nicht vorveröffentlichte Deutsche Patentanmeldung P 38 11 921.8). Für dieses Verfahren kann als Ausgangsmaterial auch ein Gemisch aus Proteinen dienen, die nur teilweise am N-Ende einen Methioninrest tragen.

In Weiterentwicklung dieses Erfindungsgedankens wurde nun gefunden, daß beliebige Proteine aus genetisch kodierten Aminosäuren hergestellt werden können, wenn man gentechnisch ein Protein herstellt, das N-terminal mindestens um Prolin verlängert ist und aus diesem Protein durch Umsetzung mit Prolin-Iminopeptidase, gegebenenfalls nach vorheriger Abspaltung anderer Aminosäuren mit Aminopeptidase-P, das gewünschte Protein freisetzt.

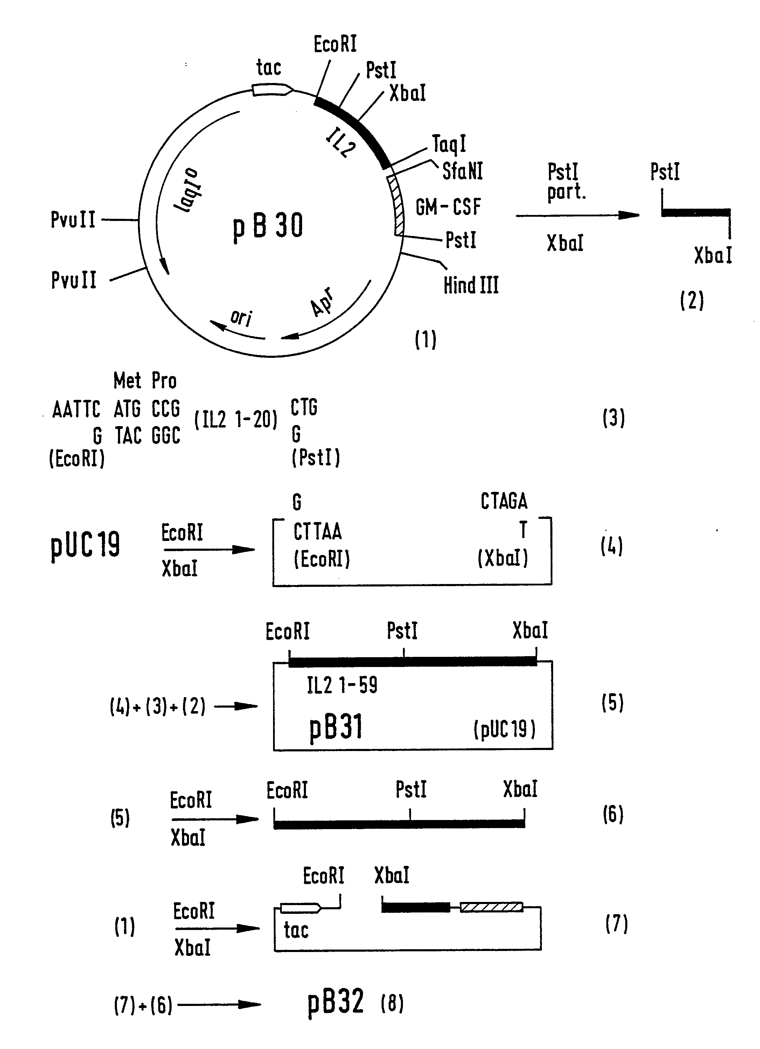

Die Figur zeigt die Konstruktion des Expressionsvektors pB32, der für ein bifunktionelles Protein aus Interleukin-2 und Granulozyten-Makrophagen-Kolonie-stimulierendem-Faktor (GM-CSF) kodiert. Dieses Protein beginnt N-terminal mit Met-Pro und stellt ein bevorzugtes Ausgangsmaterial für das erfindungsgemäße Verfahren dar.

Im folgenden werden bevorzugte Ausgestaltungen der Erfindung näher erläutert.

Als Ausgangsmaterialien für das erfindungsgemäße Verfahren können alle gentechnisch erhaltenen Proteine dienen, die N-terminal mit Prolin beginnen, also beispielsweise auch gegebenenfalls durch proteolytische Spaltung vorbehandelte Proteine, wie sie nach den Europäischen Patentanmeldungen mit den Veröffentlichungsnummern (EP-A) 0 219 781, 0 227 938 und 0 228 018 erhältlich sind. Bevorzugt werden direkt exprimierte Proteine eingesetzt, insbesondere solche, die nach dem in der Deutschen Patentanmeldung P 38 11 921.8 vorgeschlagenen Verfahren erhalten oder eingesetzt werden. Wenn als Ausgangsmaterial Proteine dienen, die vor dem Prolinrest oder vor mehreren Prolinresten noch andere Aminosäurereste, insbesondere den Methioninrest, tragen, so werden diese Reste zunächst mit Aminopeptidase-P entfernt. Geeignete Ausgangsmaterialien sind auch Gemische von gentechnisch erhaltenen Proteinen, die vor dem Prolinrest nur teilweise einen Methioninrest tragen. Daneben kommen prinzipiell auch alle anderen gentechnisch erhaltenen N-terminal zusätzlich verlängerten Proteine in Betracht, da die Aminopeptidase-P unspezifisch Aminosäuren abspaltet, die N-terminal vor Prolinresten angeordnet sind.

Die Prolin-Iminopeptidase kann in an sich bekannter Weise (Sarid et al., J. Biol. Chem. 234 (1959) 1740, 237 (1962) 2207) gewonnen werden. Vorteilhaft wird jedoch anstelle des bekannten Elektrophorese-Reinigungsschrittes eine zweite chromatographische Reinigung durchgeführt.

Die Durchführung der enzymatischen Abspaltung des Prolins erfolgt in üblicher Weise, wobei das Enzym in freier oder trägergebundener Form eingesetzt werden kann. Die Immobilisierung des Enzyms kann beispielsweise nach den Verfahren der Europäischen Patentschriften (EP-B) 0 141 223 und 0 141 224 sowie der darin genannten Literatur erfolgen.

Wenn eine enzymatische Abspaltung weiterer Aminosäuren, insbesondere von Methionin, mit Aminopeptidase-P vorangeht, so kann auch dieses Enzym in freier oder trägergebundener Form eingesetzt werden. Insbesondere beim Einsatz trägergebundener Enzyme können die beiden Enzymreaktionen kombiniert werden, indem die immobilisierten Enzyme in einem geeigneten Enzymreaktor so angeordnet werden, daß die Reaktionslösung zuerst mit der gebundenen Aminopeptidase-P und anschließend mit der gebundenen Prolin-Iminopeptidase in Kontakt kommt.

Die Erfindung erlaubt es somit, gentechnisch hergestellte Polypeptide und Proteine, insbesondere durch Direktexpression erhaltene Produkte, mit einem definierten N-Terminus zu gewinnen, wobei von milden, enzymatischen Bedingungen Gebrauch gemacht wird. Das erfindungsgemäße Verfahren ist also nicht den Nachteilen und Beschränkungen unterworfen, die chemische Abspaltungsverfahren mit sich bringen.

In den folgenden Beispielen wird die Erfindung näher erläutert. Teile und Prozentangaben beziehen sich hierbei auf das Gewicht, wenn keine anderen Angaben gemacht sind.

**Beispiel 1:**

Isolierung und Reinigung der Prolin-Iminopeptidase

Die Isolierung und Reinigung erfolgt nach Sarid et al., a.a.O., ausgehend von einem E. coli-Stamm des Typs K12-W-1361: pro⁻met⁻.

Anstelle der beschriebenen elektrophoretischen Anreicherung des Enzyms erfolgt jedoch zweckmäßig eine Chromatographie an DEAE-Cellulose (FPLC-System der Firma Pharmacia, über eine Mono Q HR55-Säule). Die enzymhaltigen Fraktionen werden gegen "Reaktionspuffer" (pro 1 ml 0,1 M 5,5-Diethylbarbitursäure und deren Natriumsalz, pH 8,6, je 50 $\mu$l 0,1 M $MnCl_2$-Lösung) dialysiert.

Sofern bei dem erfindungsgemäßen Verfahren Aminopeptidase-P eingesetzt wird, wird diese nach dem in der Deutschen Patentanmeldung P 38 11 921.8 vorgeschlagenen Verfahren isoliert:

E. coli B-Zellen werden in 50 mM Natriumphosphat-Puffer aufgeschlossen, zentrifugiert und der rohe Zellextrakt 15 Minuten bei 50°C inkubiert. Das Gemisch wird erneut zentrifugiert und eine Ammoniumsulfatfällung bei 45 % Sättigung durchgeführt. Nach Zentrifugation findet man das Enzym im Überstand. Die weitere Reinigung erfolgt entsprechend dem bekannten Verfahren.

**Beispiel 2:**

Konstruktion eines Expressionsplasmids

In der EP-A 0 288 809 (bzw. AU-A 14 661/88) ist das Expressionsplasmid pB30 vorgeschlagen, das für ein bifunktionelles Protein kodiert, bei dem auf die Aminosäurefolge von Interleukin-2 (IL 2) ein Brückenglied von 15 Aminosäuren und hierauf die Aminosäurefolge von GM-CSF folgt. Der IL 2-Anteil des Strukturgens basiert hierbei auf dem synthetischen Gen, das in der EP-A 0 163 249 (bzw. AU-A 43 070/85) beschrieben ist (Anhang). Dieses synthetische Gen enthält eine Reihe von singulären Schnittstellen für Restriktionsenzyme, wovon die für die Enzyme PstI, XbaI und SacI für die Subklonierung von Genfragmenten verwendet wurden. Die PstI-Schnittstelle befindet sich hierbei im Bereich der Aminosäuren 21/22 und die XbaI-Schnittstelle in Bereich der Aminosäuren 58-60.

Das Plasmid pB30 (1) wird partiell mit PstI und total mit XbaI verdaut und das kleine Fragment (2) isoliert, das für das IL 2-Teilfragment kodiert.

Entsprechend EP-A 0 163 249 wird das Oligonukleotid (3) synthetisiert (das der dort beschriebenen DNA-Sequenz IIa, Oligonucleotid-Bausteine Ia-If, entspricht, wobei jedoch hinter das ATG-Startcodon das für Prolin kodierende Triplett CCG eingeschoben ist).

Der handelsübliche Vektor pUC19 wird mit EcoRI und XbaI geöffnet und das große Fragment (4) mit den IL 2-Teilfragmenten (2) und (3) ligiert. Hierzu werden kompetente Zellen des E. coli-Stammes 79/02 mit dem Ligationsgemisch transformiert und auf IPTG/Xgal-Platten, die 20 mg/l Ampicillin enthalten, ausplattiert. Weiße Kolonien werden isoliert und die Plasmid-DNA durch Restriktions- und Sequenzanalyse charakterisiert. Die gewünschten Plasmide, die für die ersten 59 Aminosäuren von IL 2 kodieren, erhalten die Bezeichnung pB31 (5).

Aus dem Plasmid pB31 (5) wird das IL 2-Fragment (6) mit EcoRI und XbaI herausgespalten und anstelle des entsprechenden Fragments in das Plasmid pB30 eingesetzt. Hierzu wird pB30 (1) mit EcoRI und XbaI verdaut und das große Fragment (7) mit dem Fragment (6) ligiert, wobei das Plasmid pB32 (8) erhalten wird (auf dessen Wiedergabe in der Figur verzichtet wird, da es sich nur in der ohnehin nicht zeichnerisch dargestellten 5'-Region des IL 2-Gens unterscheidet).

Das Plasmid pB32 (8) wird in den E. coli-Stamm MM294 transformiert, amplifiziert, reisoliert und zur Expression in den E. coli-Stamm W3110 transformiert. Die Expression erfolgt nach dem in der EP-B 0 288 809 vorgeschlagenen Verfahren.

**Beispiel 3:**

Isolierung und Reinigung des bifunktionellen Proteins

Nach Abschluß der Expression werden die Zellen abzentrifugiert und in Phosphatpuffer (pH 6,5) aufgeschlossen. Das Aufschlußgemisch wird abzentrifugiert, wobei sich das bifunktionelle Protein im Sediment findet. Dieses wird mit Wasser gewaschen und anschließend in 1 %iger Desoxycholat-Lösung resuspendiert. Die Suspension wird abzentrifugiert und das Sediment erneut mit Wasser gewaschen. Es wird nun in 0,4 %iger N-Lauroylsarcosin-Natriumsalz-Lösung aufgenommen und eine Stunde geschüttelt. Nach Einstellung einer Kupfersulfat-Konzentration von 10 mM wird über Nacht bei 4° C weitergeschüttelt. Das Gemisch wird abzentrifugiert, der Überstand mit dem 6-fachen Volumen an Aceton versetzt und das ausgefallene Protein abzentrifugiert. Das Sediment wird getrocknet und mit einer solchen Menge 6 M-Guanidinium-Hydrochlorid-Lösung aufgelöst, daß eine Konzentration von 50 bis 100 mg Protein/l erreicht wird. Anschließend wird gegen einen Glycinpuffer (0,01 M Glycin, 10 mM Tris-HCl, pH 7,5) dialysiert. Nach der Dialyse wird erneut zentrifugiert und der Überstand über eine Affinitätssäule (Affi-Gel 10), die IL 2-Antikörper gebunden enthält, gegeben. Die Säule wird mit 1M NaCl in 5 mM Tris-HCl und anschließend mit 10 M Tris-HCl, pH 7,5, gewaschen, das bifunktionelle Protein mit 0,2 N Essigsäure eluiert und gegen $Mn^{2+}$-Puffer (Yaron et al., Biochemical and Biophysical Research Communications 32 (1968) 658) dialysiert.

**Beispiel 4:**

Abspaltung der N-terminalen Met-Pro-Sequenz

Das nach Beispiel 3 erhaltene bifunktionelle Protein wird auf eine Konzentration von $5 \cdot 10^{-6}$ bis $10^{-5}$ Mol eingestellt. Nun werden 0,1 bis 1 µg/ml Aminopeptidase-P zugesetzt und das Reaktionsgemisch 1 Stunde bei 37 - 40° C umgesetzt. Die Reaktion wird durch Dialyse gegen 10 mM Tris-HCl, pH 7,5, beendet. Die Reaktionspartner werden affinitätschromatographisch nach Beispiel 3 voneinander getrennt. Die das bifunktionelle Protein enthaltenden Fraktionen werden vereinigt und gegen den (in Beispiel 1 definierten) Prolin-Iminopeptidase-Reaktionspuffer dialysiert. Anschließend wird die Prolin-Iminopeptidase in einer solchen Menge zugesetzt, daß das Gewichtsverhältnis Enzym : Substrat etwa 1 : 5 beträgt. Das Reaktionsgemisch wird 8 bis 16 Stunden bei 40° C inkubiert. Dabei wird die Abspaltung des Prolins nach der Methode von Troll et al., J. Biol. Chem. 215 (1955) 655, verfolgt. Die Reaktion wird durch Zugabe einer $10^{-3}$ M $ZnCl_2$-Lösung beendet. Das Reaktionsprodukt wird über "reversed phase"-HPLC gereinigt und nach Entsalzung einer Aktivitätsbestimmung und der Proteinsequenzanalyse unterworfen. Die Sequenzanalyse zeigt einen praktisch homogenen N-Terminus.

Die Überprüfung der biologischen Aktivität im Knochenmarks-Proliferationstest ergibt Übereinstimmung mit dem in der EP-A 0 288 809 vorgeschlagenen Protein.

Anhang

<u>DNA-Sequenz I</u> aus der EP-A 0 163 249 (AU-A 43070/85)

| Triplett Nr. | | | | | | | 0 | 1 | 2 |
|---|---|---|---|---|---|---|---|---|---|
| Aminosäure | | | | | | | Met | Ala | Pro |
| Nucleotid Nr. | | | | | 1 | | | 10 | |
| Cod. Strang | | | | 5' | AA | TTC | ATG | GCG | CCG |
| nicht cod. Strang | | | | 3' | | G | TAC | CGC | GGC |

| 3 | 4 | 5 | 6 | 7 | 8 | 9 | 10 | 11 | 12 |
|---|---|---|---|---|---|---|---|---|---|
| Thr | Ser | Ser | Ser | Thr | Lys | Lys | Thr | Gln | Leu |
| | 20 | | | | 30 | | | 40 | |
| ACC | TCT | TCT | TCT | ACC | AAA | AAG | ACT | CAA | CTG |
| TGG | AGA | AGA | AGA | TGG | TTT | TTC | TGA | GTT | GAC |

| 13 | 14 | 15 | 16 | 17 | 18 | 19 | 20 | 21 | 22 |
|---|---|---|---|---|---|---|---|---|---|
| Gln | Leu | Glu | His | Leu | Leu | Leu | Asp | Leu | Gln |
| | 50 | | | | 60 | | | 70 | |
| CAA | CTG | GAA | CAC | CTG | CTG | CTG | GAC | CTG | CAG |
| GTT | GAC | CTT | GTG | GAC | GAC | GAC | CTG | GAC | GTC |

| 23 | 24 | 25 | 26 | 27 | 28 | 29 | 30 | 31 | 32 |
|---|---|---|---|---|---|---|---|---|---|
| Met | Ile | Leu | Asn | Gly | Ile | Asn | Asn | Tyr | Lys |
| | 80 | | | | 90 | | | 100 | |
| ATG | ATC | CTG | AAC | GGT | ATC | AAC | AAC | TAC | AAA |
| TAC | TAG | GAC | TTG | CCA | TAG | TTG | TTG | ATG | TTT |

| 33 | 34 | 35 | 36 | 37 | 38 | 39 | 40 | 41 | 42 |
|---|---|---|---|---|---|---|---|---|---|
| Asn | Pro | Lys | Leu | Thr | Arg | Met | Leu | Thr | Phe |
| | 110 | | | | 120 | | | 130 | |
| AAC | CCG | AAA | CTG | ACG | CGT | ATG | CTG | ACC | TTC |
| TTG | GGC | TTT | GAC | TGC | GCA | TAC | GAC | TGG | AAG |

| 43 | 44 | 45 | 46 | 47 | 48 | 49 | 50 | 51 | 52 |
|-----|-----|-----|-----|-----|-----|-----|-----|-----|-----|
| Lys | Phe | Tyr | Met | Pro | Lys | Lys | Ala | Thr | Glu |
| | 140 | | | | 150 | | | 160 | |
| AAA | TTC | TAC | ATG | CCG | AAA | AAA | GCT | ACC | GAA |
| TTT | AAG | ATG | TAC | GGC | TTT | TTT | CGA | TGG | CTT |

| 53 | 54 | 55 | 56 | 57 | 58 | 59 | 60 | 61 | 62 |
|-----|-----|-----|-----|-----|-----|-----|-----|-----|-----|
| Leu | Lys | His | Leu | Gln | Cys | Leu | Glu | Glu | Glu |
| | 170 | | | | 180 | | | 190 | |
| CTG | AAA | CAC | CTC | CAG | TGT | CTA | GAA | GAA | GAG |
| GAC | TTT | GTG | GAG | GTC | ACA | GAT | CTT | CTT | CTC |

| 63 | 64 | 65 | 66 | 67 | 68 | 69 | 70 | 71 | 72 |
|-----|-----|-----|-----|-----|-----|-----|-----|-----|-----|
| Leu | Lys | Pro | Leu | Glu | Glu | Val | Leu | Asn | Leu |
| | 200 | | | | 210 | | | 220 | |
| CTG | AAA | CCG | CTG | GAG | GAA | GTT | CTG | AAC | CTG |
| GAC | TTT | GGC | GAC | CTC | CTT | CAA | GAC | TTG | GAC |

| 73 | 74 | 75 | 76 | 77 | 78 | 79 | 80 | 81 | 82 |
|-----|-----|-----|-----|-----|-----|-----|-----|-----|-----|
| Ala | Gln | Ser | Lys | Asn | Phe | His | Leu | Arg | Pro |
| | 230 | | | | 240 | | | 250 | |
| GCT | CAG | TCT | AAA | AAT | TTC | CAC | CTG | CGT | CCG |
| CGA | GTC | AGA | TTT | TTA | AAG | GTG | GAC | GCA | GGC |

| 83 | 84 | 85 | 86 | 87 | 88 | 89 | 90 | 91 | 92 |
|-----|-----|-----|-----|-----|-----|-----|-----|-----|-----|
| Arg | Asp | Leu | Ile | Ser | Asn | Ile | Asn | Val | Ile |
| | 260 | | | | 270 | | | 280 | |
| CGT | GAC | CTG | ATC | TCT | AAC | ATC | AAC | GTT | ATC |
| GCA | CTG | GAC | TAG | AGA | TTG | TAG | TTG | CAA | TAG |

| 93 | 94 | 95 | 96 | 97 | 98 | 99 | 100 | 101 | 102 |
|-----|-----|-----|-----|-----|-----|-----|-----|-----|-----|
| Val | Leu | Glu | Leu | Lys | Gly | Ser | Glu | Thr | Thr |
| | 290 | | | | 300 | | | 310 | |
| GTT | CTG | GAG | CTC | AAA | GGT | TCT | GAA | ACC | ACG |
| CAA | GAC | CTC | GAG | TTT | CCA | AGA | CTT | TGG | TGC |

| 103 | 104 | 105 | 106 | 107 | 108 | 109 | 110 | 111 | 112 |
|---|---|---|---|---|---|---|---|---|---|
| Phe | Met | Cys | Glu | Tyr | Ala | Asp | Glu | Thr | Ala |
| | 320 | | | | 330 | | | 340 | |
| TTC | ATG | TGC | GAA | TAC | GCG | GAC | GAA | ACT | GCG |
| AAG | TAC | ACG | CTT | ATG | CGC | CTG | CTT | TGA | CGC |

| 113 | 114 | 115 | 116 | 117 | 118 | 119 | 120 | 121 | 122 |
|---|---|---|---|---|---|---|---|---|---|
| Thr | Ile | Val | Glu | Phe | Leu | Asn | Arg | Trp | Ile |
| | 350 | | | ·360 | | | | 370 | |
| ACG | ATC | GTT | GAA | TTT | CTG | AAC | CGT | TGG | ATC |
| TGC | TAG | CAA | CTT | AAA | GAC | TTG | GCA | ACC | TAG |

| 123 | 124 | 125 | 126 | 127 | 128 | 129 | 130 | 131 | 132 |
|---|---|---|---|---|---|---|---|---|---|
| Thr | Phe | Cys | Gln | Ser | Ile | Ile | Ser | Thr | Leu |
| | 380 | | | | 390 | | | 400 | |
| ACC | TTC | TGC | CAG | TCG | ATC | ATC | TCT | ACC | CTG |
| TGG | AAG | ACG | GTC | AGC | TAG | TAG | AGA | TGG | GAC |

| 133 | 134 | 135 | | | | |
|---|---|---|---|---|---|---|
| Thr | | | | | | |
| 410 | | | | | | |
| ACC | TGA | TAG | | | 3' | |
| TGG | ACT | ATC | AGC | T | 5' | |

**Ansprüche**

1. Verfahren zur Herstellung eines Proteins, dadurch gekennzeichnet, daß man gentechnisch ein Vorprodukt herstellt, das N-terminal mindestens um Prolin verlängert ist, und aus diesem Protein, gegebenenfalls nach vorheriger Abspaltung anderer Aminosäuren mit Aminopeptidase-P, durch Umsetzung mit Prolin-Iminopeptidase das gewünschte Protein freisetzt.

2. Verfahren nach Anspruch 1, dadurch gekennzeichnet, daß man ein Vorprodukt gentechnisch herstellt, das N-terminal vor dem gewünschten Protein die Sequenz Met-Pro trägt.

3. Verfahren nach Anspruch 1 oder 2, dadurch gekennzeichnet, daß das gentechnisch hergestellte Vorprodukt das Produkt einer Direktexpression ist.

4. Verfahren nach Anspruch 3, dadurch gekennzeichnet, daß das Vorprodukt durch Direktexpression in Bakterien gewonnen wird.

5. Verfahren nach Anspruch 3 oder 4, dadurch gekennzeichnet, daß man ein Gemisch einsetzt, in dem die Proteine nur teilweise vor dem Prolinrest einen Methioninrest tragen.

tac

EcoRI
PstI
XbaI

IL2

TaqI
SfaNI
GM-CSF
PstI
Hind III

PvuII
PvuII

laqIo

pB30

ori
Apr

(1)

PstI
part.
XbaI

PstI

XbaI

(2)

```
        Met Pro
AATTC  ATG CCG   (IL2 1-20)  CTG                    (3)
    G  TAC GGC               G
(EcoRI)                      (PstI)
```

```
               G                    CTAGA
pUC19   EcoRI    CTTAA                  T          (4)
        XbaI   (EcoRI)              (XbaI)
```

EcoRI        PstI        XbaI

IL2 1-59

(4)+(3)+(2) ——►   pB31        (pUC19)              (5)

EcoRI    EcoRI        PstI        XbaI
(5)   ————►                                        (6)
XbaI

EcoRI    XbaI
EcoRI
(1)   ————►   tac                                  (7)
XbaI

(7)+(6) ————►   pB32  (8)